# EUROPEAN PATENT SPECIFICATION

(11) **EP 0 525 307 B1**
(45) Date of publication and mention of the grant of the patent: **06.03.1996**
(21) Application number: 92107277.3
(22) Date of filing: 29.04.1992
(51) Int. Cl.: A61K 9/16, A61K 31/35, A61K 31/71

(54) **Stable compositions for parenteral administration and their use**
Stabile Zusammensetzungen für parenterale Verabrechung und ihre Verwendung
Compositions stables pour administration parenterale et leur utilisation

(30) Priority: 23.07.1991 US 734430
(43) Date of publication of application: 03.02.1993
(73) Proprietor: AMERICAN CYANAMID COMPANY, Wayne, NJ 07470-8426 (US)
(72) Inventor: Cady, Susan Mancini, Yardley, Pennsylvania 19067 (US); Steber, William David, Ledgewood, New Jersey 07852 (US); Hayes, Phillip Wayne, Warwick, New York 10990 (US); Doscher, Mary Ehlers, Trenton, New Jersey 08690 (US); Schwinghammer, Kurt Allen, Richboro, Pennsylvania 18954 (US)
(74) Representative: Wächtershäuser, Günter, Prof. Dr.

(56) References cited:
- EP-A- 0 257 368
- EP-A- 0 329 460
- EP-A- 0 385 106
- EP-A- 0 448 930
- DE-A- 1 808 362

## Description

Macromolecules such as LL-F28249α-λ compounds, 23-oxo and 23-imino derivatives of LL-F28249α-λ compounds, milbemycin compounds and avermectin compounds are useful for the prevention and treatment of infections and infestations caused by helminths, nematodes, acarids and endo- and ectoparasitic arthropods when parenterally administered to warm-blooded animals. Parenteral compositions are sterilized prior to administration to an animal.

EP-A-0 329 460 discloses the stabilisation of compositions comprising LL-F28249 series compounds under normal conditions of preparation, use and storage by the addition of antioxidants such as alkyl gallates.

Gamma radiation is an effective sterilization process for eliminating microbial contaminants. However, certain macromolecules such as LL-F28249α-λ compounds, 23-oxo and 23-imino derivatives of LL-F28249α-λ compounds, milbemycin compounds and avermectin compounds degrade and lose much of their biological activity when irradiated. This destructive and degradative response to gamma radiation precludes the use of gamma radiation as a means to sterilize certain macromolecule-containing compositions.

It is an object of the present invention to provide stable compositions which can be irradiated that are useful for the prevention or treatment of infections and infestations caused by helminths, nematodes, acarids and endo- and ectoparasitic arthropods when parenterally administered to warm-blooded animals.

It is also an object of this invention to provide a method for introducing and maintaining levels of LL-F28249α-λ compounds, 23-oxo and 23-imino derivatives of LL-F28249α-λ compounds, milbemycin compounds and avermectin compounds in the blood of warm-blooded animals for extended periods of time.

The present invention relates to stable microsphere compositions which can be irradiation sterilized for parenteral administration. The compositions comprise on a weight basis 20% to 95% of a fat or wax or mixture thereof, 1% to 50% of a LL-F28249α-λ compound, a 23-oxo or 23-imino derivative of a LL-F28249α-λ compound, a milbemycin compound or an avermectin compound, 1% to 30% of an oil, semi-soft fat, fatty acid derivative or mixtures thereof and 0.05% to 10% of an antioxidant.

Surprisingly, it has been found that the microsphere compositions of the invention can be sterilized by gamma radiation without degradation of its biological activity.

The stable microsphere compositions of this invention are parenterally administered by dispersion in a pharmaceutically and pharmacologically acceptable liquid vehicle. The invention is also a slow release composition of the microspheres dispersed in a pharmaceutically and pharmacologically acceptable liquid vehicle. The invention also includes a method for introducing and maintaining blood levels of a LL-F28249α-λ compound, a 23-oxo or 23-imino derivative of a LL-F28249α-λ compound, a milbemycin compound or an avermectin compound in warm-blooded animals for an extended period of time.

Preferred stable microsphere compositions of the invention comprise on a weight basis 50% to 90% of a fat or wax or mixture thereof, 5% to 25% of a LL-F28249α-λ compound, a 23-oxo or 23-imino derivative of a LL-F28249α-λ compound, a milbemycin compound or an avermectin compound, 1% to 20% of an oil, semi-soft fat, fatty acid derivative or mixtures thereof and 1% to 5% of an antioxidant.

The compounds designated LL-F28249α-λ are (collectively) isolates from the fermentation broth of the microorganism Streptomyces cyaneogriseus subspecies noncyanogenus, deposited in the NRRL under deposit accession No. 15773.

The LL-F28249α-λ compounds are represented by the following structural formula: 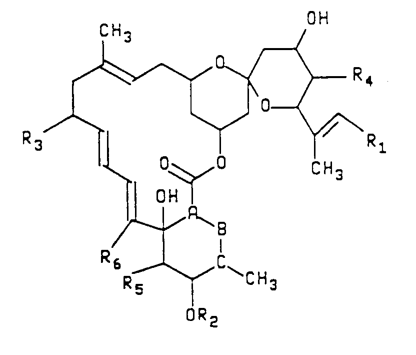
LL-F28249α-λ

| LL-F28249 | R₁ | R₂ | R₃ | R₄ | R₅ | R₆ | R₅+R₆ | A-B | B-C |
|---|---|---|---|---|---|---|---|---|---|
| alpha | CH(CH₃)2 | H | CH₃ | CH₃ | | | -O-CH₂- | CH-CH | CH=C |
| beta | CH₃ | H | CH₃ | CH₃ | | | -O-CH₂- | CH-CH | CH=C |
| gamma | CH₃ | CH₃ | CH₃ | CH₃ | | | -O-CH₂ | CH-CH | CH=C |
| delta | CH₃ | CH₃ | CH₃ | CH₃ | OH | CH₂OH | | CH-CH | CH=C |
| epsilon | CH(CH₃)2 | H | H | CH₃ | | | -O-CH₂ | CH-CH | CH=C |
| zeta | CH₂CH₃ | H | CH₃ | CH₃ | | | -O-CH₂ | CH-CH | CH=C |
| eta | CH(CH₃)2 | H | CH₃ | CH₃ | | | -O-CH₂ | C=CH | CH-CH |
| theta | CH(CH₃)2 | H | CH₃ | CH₂CH₃ | | | -O-CH₂ | CH-CH | CH=C |
| iota | CH(CH₃)2 | H | CH₂CH₃ | CH₃ | | | -O-CH₂- | CH-CH | CH=C |
| kappa | CH₃ | CH₃ | CH₃ | CH₃ | H | CH₃ | | CH-CH | CH=C |
| lambda | CH(CH₃)2 | CH₃ | CH₃ | CH₃ | | | -O-CH₂ | CH-CH | CH=C |

The 23-oxo and 23-imino derivatives of LL-F28249α-λ compounds, useful in the stable microsphere compositions of this invention, are disclosed in United States Patent No. 4,916,154 which is incorporated herein by reference thereto.

A preferred LL-F28249α-λ compounds and 23-imino derivative of a LL-F28249α-λ compound useful in the microsphere compositions of this invention have the following structural formulas:
F28249α 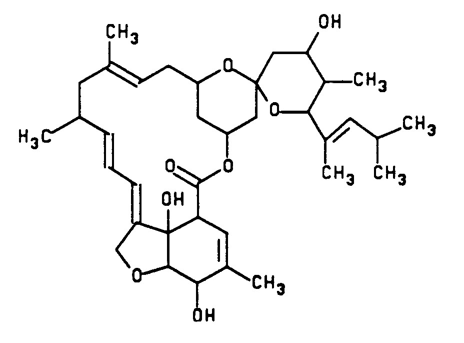
and
23-(O-methyloxime)-F28249α 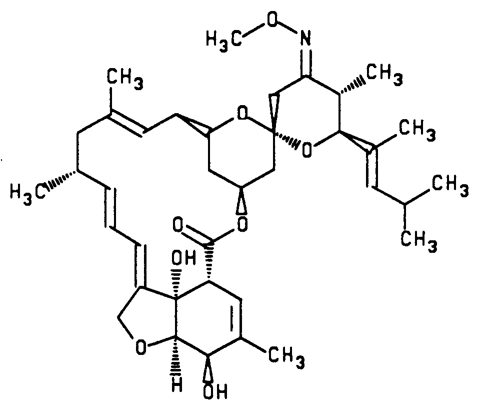
Waxes and fats which are suitable for use in the compositions of this invention in general have melting points higher than 40°C. The wax of the invention may be defined as set forth in Hawley's The Condensed Chemical Dictionary, Eleventh Edition, as a low-melting organic mixture or compound of high molecular weight, solid at room temperature and generally similar in composition to fats and oils except that it contains no glycerides. Some are hydrocarbons; others are esters of fatty acids and alcohols. These compounds include saturated or unsaturated long chain C₁₀-C₂₄ fatty acids, alcohols, esters, salts, ethers or mixtures thereof. They are classed among the lipids. Waxes are thermoplastic, but since they are not high polymers, they are not considered in the family of plastics. Common properties are water repellency; smooth texture; nontoxicity; freedom from objectionable odor and color. They are combustible and have good dielectric properties. They are soluble in most organic solvents and are insoluble in water. The major types are as follows:
I. Natural
   1. Animal (beeswax, lanolin, shellac wax, Chinese insect wax)
   2. Vegetable (carnauba, candelilla, bayberry, sugar cane)
   3. Mineral (a) Fossil or earth waxes (ozocerite, ceresin, montan)
      (b) petroleum waxes (paraffin, microcrystalline) (slack or scale wax)
II. Synthetic
   1. Ethylenic polymers and polyol ether-esters ("Carbowax")
   2. Chlorinated naphthalenes ("Halowax")
   3. Hydrocarbon type via Fischer-Tropsch synthesis

The fat of the invention may be defined as set forth in Hawley's The Condensed Chemical Dictionary, Eleventh Edition, as a glyceryl ester of higher fatty acids such as stearic and palmitic. Such esters and their mixtures are solids at room temperatures and exhibit crystalline structure. Lard and tallow are examples. The term "fat" usually refers to triglycerides specifically, whereas "lipid" is all-inclusive.

The fat is preferably composed of mono-, di- or triglyceryl esters of long chain C₁₀-C₂₄ fatty acids. The mono-, di- or triglycerides are composed predominantly of stearates, palmitates, laurates, linoleates, linolenates, oleates, and residues or mixtures thereof, having melting points greater than 50^{o}C are most preferred. Glyceryl tristearate is a most preferred fat. Additionally, lipophilic salts of fatty acids such as magnesium stearate and the like are also suitable.

The oil, semi-soft fat or fatty acid derivative of the invention are agents which are soluble in the molten hard fat and which accelerate physical transformation of the hard fat crystal from less stable forms to more stable forms at or near room temperature after the molten hard fat or wax has been atomized. Preferably, the oil or semi-soft fat may include mixtures or relatively pure forms of mono-, di- or triglyceryl esters with short to medium fatty acid chain lengths, that is C₂ to C₁₈. Semi-soft fats refer to glyceryl esters having melting points at or near room temperature. Fatty acid derivatives include short and medium chain length fatty acids, alcohols, esters, ethers, salts or mixtures thereof. Glyceride oils and semi-soft fats are particularly suitable because they are physiological constituents of the body and are biocompatible and biodegradable.

The antioxidant of the invention may be defined as set forth in Hawley's The Condensed Chemical Dictionary, Eleventh Edition, as an organic compound added to rubber, natural fats and oils, food products, gasoline, and lubricating oils to retard oxidation, deterioration, rancidity, and gum formation, respectively. Rubber antioxidants are commonly of an aromatic amine type, such as di-β-naphthyl-p-phenylene-diamine and phenyl-β-naphthylamine. Many antioxidants are substituted phenolic compounds (butylated hydroxyanisole, di-tertbutyl-p-cresol, and propyl gallate). Food antioxidants are effective in very low concentration and not only retard rancidity but protect the nutritional value by minimizing the breakdown of vitamins and essential fatty acids.

Surprisingly, the antioxidant of the invention allows the present microsphere compositions to be sterilized with gamma radiation and maintian shelf life without significant loss of biological activity. In the absence of an antioxidant, macromolecules such as LL-F28249α-λ compounds, 23-oxo and 23-imino derivatives of LL-F28249α-λ compounds, milbemycin compounds and avermectin compounds degrade and lose much of their biological activity when irradiated. Antioxidants suitable for use in the microsphere compositions of the invention include butylated hydroxytoluene, butylated hydroxyanisole, tert-butylhydroxyquinone, propyl gallate, alpha tocopherol, 6-ethoxy-1,2-dihydro-2,2,4-trimethylquinoline and the like with butylated hydroxytoluene being a preferred antioxidant.

The stable microspheres of the invention are dispersed in a pharmaceutically and pharmacologically acceptable liquid to obtain a slow release composition for parenteral administration. The vehicle may be aqueous buffered systems or oil systems. The oil system may be derived from animal or vegetable sources or be synthetic. Preferred vehicles include neutral mono-, di- or triglyceride liquid or mixtures thereof. A neutral oil is one containing no residual acid. Vehicles suitable for use in the compositions of this invention include aqueous systems such as buffered salines; organic solvents such as glycols and alcohols; and water immiscible liquids such as oils, depending upon the solubility of the active ingredient being administered.

Excipients such as surfactants, salts, buffers or mixtures thereof may be included in the compositions of the invention. The amounts of said excipients suitable for use in the invention range from about 0.1% to 20% on a weight basis.

Blood levels of the LL-F28249α-λ compounds, 23-oxo and 23-imino derivatives of LL-F28249α-λ compounds, milbemycin compounds and avermectin compounds may be obtained and maintained for extended periods of time by injecting animals with the compositions of the invention in a suitable vehicle.

Maintained blood levels of the active compounds are associated with the protection or treatment of warm-blooded animals against infections and infestation by helminths, nematodes, acarids and endo- and ectoparasitic arthropods. Maintaining the blood levels is an indication of the slow release of the active ingredient. The invention includes the use of the compositions herein to introduce and maintain levels of LL-F28249α-λ compounds, 23-oxo and 23-imino derivatives of LL-F28249α-λ compounds, milbemycin compounds and avermectin compounds in the blood stream of animals.

When parenterally administered, the compositions of this invention are highly effective for protecting or treating warm-blooded animals such as dogs, cats, cattle, sheep, horses, swine, goats, poultry and the like against infection and infestation by helminths, nematodes, acarids and endo- and ectoparasitic arthropods.

Helminthiasis is a widespread disease found in many animals and is responsible for significant economic losses throughout the world. Among the helminths most frequently encountered are the group of worms referred to as nematodes. The nematodes are found in the intestinal tract, heart, lungs, blood vessels and other body tissues of animals and are a primary cause of anemia, weight loss and malnutrition in the infected animals. They do serious damage to the walls and tissue of the organs in which they reside and, if left untreated, may result in death to the infected animals.

The nematodes most commonly found to be the infecting agents of ruminants include Haemonchus and Ostertagia generally found in the abomasum; Cooperia, Trichostrongylus and Nematodirus generally found in the intestinal tract, and Dictyocaulus found in the lungs. In non-ruminant animals important nematodes include Toxocara and Ancylostoma in the intestine and Dirofilaria in the heart of dogs; Ascaris in the intestine of swine, Ascaridia and Heterakis in the intestine of poultry; and large and small strongyles in equines. Treatment of animals to prevent infestation thereof by the above nematodes or to reduce or control the proliferation of these infecting agents in animals is thus an important and desirable advantage of the present invention.

Besides controlling helminths and nematodes, the present invention also controls arthropod endoparasitic infestations such as cattle grub and ectoparasitic infestations such as psoroptic mange.

The microspheres of the invention may be prepared by incorporating the active ingredient, antioxidant and other excipients with a molten fat, wax or mixture thereof admixing the oil, semi-soft fat, fatty acid derivative or mixtures thereof and then forming microspheres of the resulting mixture by a variety of techniques such as emulsifying or atomizing the mixture or by processing the mixture of ingredients and molten fat, wax or mixture thereof mechanically and cooling, for example utilizing a centrifugal disc. Alternatively, the mixture of active ingredients, antioxidants, excipients and fat, waxes and mixtures thereof and oil, may be cooled to give a solid which may then be processed by procedures such as milling, grinding and the like. The microspheres, preferably fat microspheres, may be up to 1,000 microns in diameter, with a weight average size range of about 25 microns to 300 microns being preferred for parenteral administration.

In order to facilitate a further understanding of the invention, the following examples are presented primarily for the purpose of illustrating more specific details thereof. The invention is not to be deemed limited thereby except as defined in the claims.

### EXAMPLE 1

### Preparation of microspheres for the parenteral admimistration of 23-(O-methyloxime)-F28249α

Glyceryl tristearate (72.8 g) and triglyceride oil (8.7 g) are heated to 85^{o}-90^{o}C in a beaker on a hot plate, 23(O-methyloxime)-F28249α (12.0 g) and butylated hydroxytoluene (1.1 g) are added with constant stirring until the 23-(O-methyloxime)-F28249α and butylated hydroxytoluene dissolve. This solution is then poured into a feed bowl of a laboratory spray priller. The feed bowl, feed lines, air supply lines and a two fluid spray atomizing nozzle are maintained at 85^{o}-90^{o}C with hot air. Fluid feed to the nozzle is assisted with a 1 psi pressure head and the atomizing air pressure is 10 psi. The atomized mixture is collected from the outlet of the prilling chamber with a cyclone separator. The microspheres are then sieved to remove microspheres larger than 180 microns. Those microspheres which are larger than 180 microns are collected for recycling.

The microspheres obtained using the above procedure have the following composition.

| | % w/w |
|---|---|
| 23-(O-methyloxime)-F28249α | 12.0 |
| glyceryl tristearate | 78.2 |
| triglyceride oil | 8.7 |
| butylated hydroxytoluene (BHT) | 1.1 |

### EXAMPLE 2

### Stability of microspheres to irradiation

The microsphere compositions listed below are placed in 20 mL serum vials, two of which are flushed with dry nitrogen gas to remove oxygen. The vials are then closed with elastomeric septums and crimped aluminum caps. Next, the microspheres are irradiated at ambient temperature with cobalt 60(gamma radiation) with the dosages indicated in Table I below. The microspheres are extracted into acetonitrile/water (1:1 and analyzed for 23-(O-methyloxime)-F28249α by high performance liquid chromatography. The results of this experiment are summarized below in Table I.

| Microsphere Compositions | | |
|---|---|---|
| Ingredient | A¹ (% w/w) | B² (% w/w) |
| 23-(O-methyloxime)-F28249α | 12.0 | 12.0 |
| glyceryl tristearate | 79.2 | 78.2 |
| medium chain triglyceride oil | 8.8 | 8.7 |
| butylated hydroxytoluene | 0 | 1.1 |

| | | |
|---|---|---|
| ¹Prepared by the procedure of Example 1, except that butylated hydroxytoluene is not utilized | | |
| ² Microsphere composition prepared in Example 1 | | |

**TABLE I**

| Percent Recovery of 23-(O-Methyloxime)-F28249α From Microsphere Compositions After Irradiation | | | | |
|---|---|---|---|---|
| Exposure (mrad) | Composition | | | |
| | A | | B | |
| | Air | Nitrogen | Air | Nitrogen |
| 0 | 94.5 | 94.5 | 96.5 | 96.5 |
| 0.55 | 68.5 | 92.5 | 95.0 | 96.5 |
| 2.1 | 27.5 | 90.5 | 96.0 | 95.0 |

This experiment demonstrates that the active ingredient present in microspheres without BHT degrades when irradiated with gamma radiation in either air or nitrogen atmospheres and that the active ingredient is stable when BHT is present in the formulation.

### EXAMPLE 3

### Sustained release of compositions of the invention in dogs

Dogs are divided into groups of three or four animals each. Throughout the test, all dogs are fed the same ration. The dogs are weighed and injected with a composition containing the microsphere composition of example 1 (1 g) mixed with a 2.5% methylcellulose in normal saline solution (2 mL) in amounts required to obtain the desired dose of active ingredient per kg of body weight. 23-(O-methyloxime)-F28249α levels in the blood of the dogs is determined by HPLC techniques periodically. The results of this experiment, which are summarized in Table II below, demonstrate the effectiveness of the compositions of the invention for introducing and maintaining 23-(O-methyloxime)-F28249α levels in the blood.

**TABLE II**

| Average Plasma 23-(O-methyloxime)-F28249α Concentration (ppb by HPLC) For Dog Experiments | | |
|---|---|---|
| Time (days) | Dose (mg/kg) | |
| | 5¹ | 1.7² |
| 1 | 111 | 39 |
| 3 | 197 | 80 |
| 8 | 340 | 100 |
| 15 | 133 | 50 |
| 21 | 80 | 29 |
| 28 | 68 | 21 |
| 35 | 51 | 14 |
| 42 | 33 | 10 |
| 49 | 20 | 7 |

| | | |
|---|---|---|
| ¹three dogs | | |
| ²four dogs | | |

## Claims (Claims for the following Contracting State(s): AT, BE, CH, DE, DK, FR, GB, IT, LI, LU, NL, PT, SE)

1. A microsphere composition characterized by, on a weight basis, 20% to 95% of a fat or wax or mixture thereof, 1% to 50% of a compound selected from the group consisting of a LL-F28249α-λ, a 23-oxo or 23-imino derivative of a LL-F28249α-λ a milbemycin and an avermectin, 1% to 30% of an oil, semi-soft fat, fatty acid derivative or mixtures thereof, 0.05% to 10% of an antioxidant, and 0% to 20% of a surfactant, salt, buffer or mixture thereof.

2. The microsphere composition according to claim 1 wherein the compound is selected from the group consisting of 23-(O-methyloxime)-F28249α and LL-F28249α, the fat is glyceryl tristearate, the oil is a neutral triglyceride oil and the antioxidant is butylated hydroxytoluene and wherein the weight average particle size of the microsphere composition is in a range of 25 microns to 300 microns.

3. The microsphere composition according to claim 1 for parenteral administration and slow release further characterized by a pharmaceutically and pharmacologically acceptable liquid vehicle in which the microspheres are dispersed.

4. A process for preparing microsphere compositions for parental administration to warm-blooded animals characterized by:
(a) mixing 1% to 50% of a compound selected from the group consisting of a LL-F28249α-λ, a 23-oxo or 23-imino derivative of a LL-F28249α-λ, a milbemycin and an avermectin; 0.05% to 10% of an antioxidant; and 0% to 20% of a surfactant, salt, buffer or mixture thereof; with 20% to 95% of a molten fat, wax or mixture thereof;
(b) admixing 1% to 30% of an oil, semi-soft fat, fatty acid derivatives or mixtures thereof; to form a molten mixture;
(c) forming microspheres by emulsifying, atomizing, or centrifugal discing the molten mixture; or cooling the molten mixture and milling or grinding the cooled mixture to form the microspheres.

5. The process according to claim 4 wherein step (a) and step (b) occur simultaneously.

## Claims (Claims for the following Contracting State(s): ES, GR)

1. A process for preparing microsphere compositions for parental administration to warm-blooded animals characterized by:
(a) mixing 1% to 50% of a compound selected from the group consisting of a LL-F28249α-λ, a 23-oxo or 23-imino derivative of a LL-F28249α-λ, a milbemycin and an avermectin; 0.05% to 10% of an antioxidant; and 0% to 20% of a surfactant, salt, buffer or mixture thereof; with 20% to 95% of a molten fat, wax or mixture thereof;
(b) admixing 1% to 30% of an oil, semi-soft fat, fatty acid derivatives or mixtures thereof; to form a molten mixture;
(c) forming microspheres by emulsifying, atomizing, or centrifugal discing the molten mixture; or cooling the molten mixture and milling or grinding the cooled mixture to form the microspheres.

2. The process according to claim 1 wherein step (a) and step (b) occur simultaneously.

3. The process according to claim 1 wherein the compound is selected from the group consisting of 23-(O-methyloxime)-F28249α and LL-F28249α, the fat is glyceryl tristearate, the oil is a neutral triglyceride oil and the antioxidant is butylated hydroxytoluene and wherein the weight average particle size of the microsphere composition is in a range of 25 microns to 300 microns.

4. The process according to claim 1 which is further characterized by dispersing the microspheres in a pharmaceutically and pharmacologically acceptable liquid vehicle for parenteral administration and slow release of said microspere composition.

## Patentansprüche (Patentansprüche für folgende(n) Vertragsstaat(en): AT, BE, CH, DE, DK, FR, GB, IT, LI, LU, NL, PT, SE)

1. Mikrokugel-Zusammensetzung, gekennzeichnet durch, auf Gewichtsgrundlage, 20 % bis 95 % eines Fettes oder Wachses oder Gemisches davon, 1 % bis 50 % einer Verbindung, ausgewählt aus der Gruppe, bestehend aus einem LL-F28249α-λ, einem 23-Oxo- oder 23-Iminoderivat eines LL-F28249α-λ, einem Milbemycin und einem Avermectin, 1 % bis 30 % eines Öls, halbweichen Fettes, Fettsäurederivats oder Gemischen davon, 0,05 % bis 10 % eines Antioxidans und 0 % bis 20 % eines Tensids, Salzes, Puffers oder Gemisches davon.

2. Mikrokugel-Zusammensetzung nach Anspruch 1, wobei die Verbindung ausgewählt ist aus der Gruppe, bestehend aus 23-(O-Methyloxim)-F28249α und LL-F28249α, wobei das Fett Glyceryltristearat ist, das Öl ein neutrales Triglyceridöl ist und das Antioxidans butyliertes Hydroxytoluol ist und wobei die gewichtsmittlere Teilchengröße der Mikrokugel-Zusammensetzung im Bereich von 25 µm bis 300 µm liegt.

3. Mikrokugel-Zusammensetzung nach Anspruch 1 zur parenteralen Verabreichung und langsamen Abgabe; außerdem gekennzeichnet durch ein pharmazeutisch und pharmakologisch verträgliches, flüssiges Vehiculum, in dem die Mikrokugeln dispergiert sind.

4. Verfahren zur Herstellung von Mikrokugel-Zusammensetzungen zur parenteralen Verabreichung an Warmblüter, gekennzeichnet durch:
(a) Vermischen von 1 % bis 50 % einer Verbindung, ausgewählt aus der Gruppe, bestehend aus einem LL-F28249α-λ, einem 23-Oxo- oder 23-Iminoderivat eines LL-F28249α-λ, einem Milbemycin und einem Avermectin; 0,05 % bis 10 % eines Antioxidans und 0 % bis 20 % eines Tensids, Salzes, Puffers oder Gemisches davon; mit 20 % bis 95 % eines geschmolzenen Fettes, Wachses oder Gemisches davon;
(b) Anmischen von 1 % bis 30 % eines Öls, halbweichen Fettes, Fettsäurederivaten oder Gemischen davon unter Herstellen eines geschmolzenen Gemisches;
(c) Formen von Mikrokügelchen durch Emulgation, Zerstäuben oder Schleuderscheiben-Zerstäubung des geschmolzenen Gemisches oder Kühlen des geschmolzenen Gemisches und Vermahlen oder Zerkleinern des gekühlten Gemisches unter Herstellen von Mikrokugeln.

5. Verfahren nach Anspruch 4, wobei Schritt (a) und Schritt (b) gleichzeitig stattfinden.

## Patentansprüche (Patentansprüche für folgende(n) Vertragsstaat(en): ES, GR)

1. Verfahren zur Herstellung von Mikrokugel-Zusammensetzungen zur parenteralen Verabreichung an Warmblüter, gekennzeichnet durch:
(a) Vermischen von 1 % bis 50 % einer Verbindung, ausgewählt aus der Gruppe, bestehend aus einem LL-F28249α-λ, einem 23-Oxo- oder 23-Iminoderivat eines LL-F28249α-λ, einem Milbemycin und einem Avermectin; 0,05 % bis 10 % eines Antioxidans und 0 % bis 20 % eines Tensids, Salzes, Puffers oder Gemisches davon; mit 20 % bis 95 % eines geschmolzenen Fettes, Wachses oder Gemisches davon;
(b) Anmischen von 1 % bis 30 % eines Öls, halbweichen Fettes, Fettsäurederivaten oder Gemischen davon unter Herstellen eines geschmolzenen Gemisches;
(c) Formen von Mikrokügelchen durch Emulgation, Zerstäuben oder Schleuderscheiben-Zerstäubung des geschmolzenen Gemisches oder Kühlen des geschmolzenen Gemisches und Vermahlen oder Zerkleinern des gekühlten Gemisches unter Herstellen von Mikrokugeln.

2. Verfahren nach Anspruch 1, wobei Schritt (a) und Schritt (b) gleichzeitig stattfinden.

3. Verfahren nach Anspruch 1, wobei die Verbindung ausgewählt ist aus der Gruppe, bestehend aus 23-(O-Methyloxim)-F28249α und LL-F28249α, wobei das Fett Glyceryltristearat ist, das Öl ein neutrales Triglyceridöl ist und das Antioxidans butyliertes Hydroxytoluol ist und wobei die gewichtsmittlere Teilchengröße der Mikrokugel-Zusammensetzung im Bereich von 25 µm bis 300 µm liegt.

4. Verfahren nach Anspruch 1, außerdem gekennzeichnet durch Dispergieren der Mikrokugeln in einem pharmazeutisch und pharmakologisch verträglichen flüssigen Vehiculum zur parenteralen Verabreichung und langsamen Abgabe der Mikrokugel-Zusammensetzung.

## Revendications (Revendications pour l'(les) Etat(s) contractant(s) suivant(s): AT, BE, CH, DE, DK, FR, GB, IT, LI, LU, NL, PT, SE)

1. Composition en microsphères caractérisée en ce qu'elle contient en poids 20 % à 95 % d'une graisse ou d'une cire ou d'un de leurs mélanges, 1 % à 50 % d'un composé choisi parmi un LL-F28249α à λ, un dérivé 23-oxo ou 23-imino d'un LL-F28249α à λ, une milbémycine et une avermectine, 1 % à 30 % d'une huile, d'une graisse semi-molle, d'un dérivé d'acide gras ou d'un de leurs mélanges, 0,05 % à 10 % d'un antioxydant et 0 % à 20 % d'un agent tensio-actif, d'un sel, d'un tampon ou d'un de leurs mélanges.

2. Composition en microsphères selon la revendication 1, où le composé est choisi parmi la 23-(O-méthyloxime)-F28249α et le LL-F28249α, la graisse est le tristéarate de glycéryle, l'huile est une huile triglycéridique neutre et l'antioxydant est l'hydroxytoluène butylé et où la moyenne en poids de la taille des particules de la composition en microsphères est dans une gamme de 25 µm à 300 µm.

3. Composition en microsphères selon la revendication 1 pour l'administration parentérale et la libération lente, caractérisée de plus en ce que les microsphères sont dispersées dans un véhicule liquide pharmaceutiquement et pharmacologiquement acceptable.

4. Procédé pour préparer des compositions en microsphères pour l'administration parentérale à des animaux à sang chaud, caractérisé par :
(a) le mélange de 1 % à 50 % d'un composé choisi parmi un LL-F28249α à λ, un dérivé 23-oxo ou 23-imino d'un LL-F28249α à λ, une milbémycine et une avermectine ; 0,05 % à 10 % d'un antioxydant ; et 0 % à 20 % d'un agent tensio-actif, d'un sel, d'un tampon ou d'un de leurs mélanges ; avec 20 % à 95 % d'une cire, d'une graisse ou d'un de leurs mélanges à l'état fondu ;
(b) le mélange de 1 % à 30 % d'une huile, d'une graisse semi-molle, d'un dérivé d'acide gras ou d'un de leurs mélanges ; pour former un mélange fondu ;
(c) la formation de microsphères par émulsification, atomisation ou traitement avec un disque centrifuge du mélange fondu ; ou le refroidissement du mélange fondu et la division ou le broyage du mélange fondu pour former les microsphères.

5. Procédé selon la revendication 4, où l'étape (a) et l'étape (b) ont lieu simultanément.

## Revendications (Revendications pour l'(les) Etat(s) contractant(s) suivant(s): ES, GR)

1. Procédé pour la préparation de compositions en microsphères pour administration parentérale à des animaux à sang chaud, caractérisé en ce que :
(a) on mélange de 1 % à 50 % d'un composé choisi parmi un LL-F28249α à λ, un dérivé 23-oxo ou 23-imino d'un LL-F28249α à λ, une milbémycine et une avermectine; 0,05 % à 10 % d'un antioxydant; et 0 % à 20 % d'un agent tensio-actif, d'un sel, d'un tampon ou d'un de leurs mélanges; avec 20 % à 95 % d'une cire, d'une graisse ou d'un de leurs mélanges à l'état fondu;
(b) on mélange de 1 % à 30 % d'une huile, d'une graisse semi-molle, d'un dérivé d'acide gras ou d'un de leurs mélanges, pour former un mélange fondu;
(c) on forme des microsphères par émulsification atomisation ou traitement avec un disque centrifuge du mélange fondu; ou le refroidissement du mélange fondu et la division ou le broyage du mélange fondu pour former les microsphères.

2. Procédé selon la revendication 1, où l'étape (a) et l'étape (b) ont lieu simultanément.

3. Procédé selon la revendication 1, où le composé est choisi parmi la 23-(O-méthyloxime)-F28249α et le LL-F28249α, la grasse est le tristéarate de glycéryle, l'huile est une huile triglycéridique neutre et l'antioxydant est l'hydroxytoluène butylé et où la moyenne en poids de la taille des particules de la composition en microsphères est dans une gamme de 25 µm à 300 µm.

4. Procédé selon la revendication 1, caractérisé de plus en ce que l'on disperse les microsphères dans un véhicule liquide pharmaceutiquement et pharmacologiquement acceptable pour administration parentérale et libération lente de ladite composition en microsphères.
